# EUROPÄISCHE PATENTSCHRIFT

(11) **EP 0 457 139 B1**
(45) Veröffentlichungstag und Bekanntmachung des Hinweises auf die Patenterteilung: **24.01.1996**
(21) Anmeldenummer: 91107246.0
(22) Anmeldetag: 04.05.1991
(51) Int. Cl.: C07D 233/58

(54) **Verfahren zur Herstellung von N-substituierten Imidazolen**
Process for the preparation of N-substituted imidazoles
Procédé pour la préparation d'imidazoles N-substitués

(30) Priorität: 15.05.1990 DE 4015535
(43) Veröffentlichungstag der Anmeldung: 21.11.1991
(73) Patentinhaber: BASF Aktiengesellschaft, D-67063 Ludwigshafen (DE)
(72) Erfinder: Ebel, Klaus, Dr., W-6704 Mutterstadt (DE)

(56) Entgegenhaltungen:
- DE-A- 2 442 706
- JOURNAL OF THE AMERICAN CHEMICAL SOCIETY Bd. 93, Nr. 24, 1971, Seiten 6584 - 6591; T. MAUGH II ET AL: 'Hydrolysis and aminolysis of O-acylhydroxyquinolines. Intracomplex general base-catalyzed aminolysis.'
- LIEBIGS ANNALEN DER CHEMIE Nr. 6, 1983, Seiten 1078 - 1080; B. PILARSKI: 'A new method for N-alkylation of imidazoles and benzimidazoles'
- CHEMICAL ABSTRACTS, vol. 70, no. 3, 20. Januar 1969, Columbus, Ohio, US; abstract no. 11699F, Seite 288; Spalte 2; & JP-A-68 012 354
- Comprehensive Organic Chemistry, (Barton and Ollis), Band 1, S. 544-546

## Beschreibung

Die vorliegende Erfindung betrifft ein neues Verfahren zur Herstellung von N-substituierten Imidazolen durch Umsetzung von Imidazolen, die am Stickstoff unsubstituiert sind (ein Wasserstoffatom tragen), mit Halogeniden und wäßrigen Hydroxid-Lösungen in Gegenwart von N-substituierten Imidazolen.

Aus Liebigs Ann. Chem. (1983), 1078-1080 ist ein Verfahren zur Alkylierung von Imidazolen zur Synthese von 1-Alkylimidazolen bekannt. Bei diesem Verfahren werden Imidazol mit Methyliodid bzw. 2-Methylimidazol mit Ethyliodid direkt mit 50 %iger Natronlauge als Reagens zu 1-Methylimidazol oder 1-Ethyl-2-methylimidazol umgesetzt.

Dieses Verfahren hat für die Anwendung im technischen Maßstab jedoch Nachteile wie die Exothermie der Reaktion, die erst nach einer Latenzzeit einsetzt und dann im größeren Mapstab schwer zu kontrollieren ist sowie die unbefriedigenden Ausbeuten.

Der vorliegenden Erfindung lag daher die Aufgabe zugrunde, den zuvor genannten Nachteilen abzuhelfen.

Demgemäß wurde ein neues und verbessertes Verfahren zur Herstellung von N-substituierten Imidazolen der allgemeinen Formel I 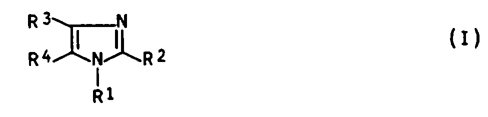 in der die Substituenten
- R1: C₁- bis C₂₀-Alkyl, Allyl, 2-Buten-1-yl, 4-Buten-2-yl, 4-Buten-1-yl, 2-Penten-1-yl, 2,2-Dimethylpenten-1-yl, Propinyl, 1,1-Dimethylpropinyl, 1-Methylpropinyl, 1-Butinyl, 2-Butinyl und 4,4-Dibutyl-2-in-1-yl, C₂- bis C₂₀-Alkoxyalkyl, C₃- bis C₂₀-Alkenyloxyalkyl, C₃- bis C₁₂-cycloalkyl, C₄- bis C₂₀-Cycloalkyl-alkyl, gegebenenfalls durch C₁- bis C₈-Alkyl, C₁- bis C₈-Alkoxy, Halogen, C₁- bis C₄-Halogenalkyl, C₁- bis C₄-Halogenalkoxy oder Phenoxy substituiertes C₇- bis C₂₀-Arylalkyl,
- R,R³,R⁴: unabhängig voneinander Wasserstoff, C₁- bis C₂₀-Alkyl, C₂- bis C₂₀-Alkoxyalkyl, C₃- bis C₁₂-Cycloalkyl, Halogen, gegebenenfalls durch C₁- bis C₈-Alkyl, C₁- bis C₈-Alkoxy, Halogen, C₁- bis C₄-Halogenalkyl, C₁- bis C₄-Halogenalkoxy oder Phenoxy, substituiertes Aryl oder C₇- bis C₂₀-Arylalkyl oder R³ und R⁴ gemeinsam eine gegebenenfalls durch C₁- bis C₈-Alkyl, C₁- bis C₈-Alkoxy und/oder Halogen ein- bis zweifach substituierte (CH₂)ₙ- oder (CH=CH)ₘ-Gruppe, in der n für 1 bis 6 und m für 1 bis 3 steht,
bedeuten, durch Umsetzung von Imidazolen der allgemeinen Formel II 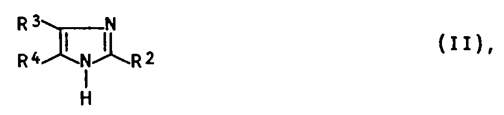
in der R²_{,} R³ und R⁴ die obengenannten Bedeutungen haben, mit Halogeniden der allgemeinen Formel III

R¹-X (III),

in der X für Chlor, Brom oder Jod steht und R¹ die obengenannten Bedeutungen hat, in wäßrigen Hydroxid-Lösungen bei Temperaturen von 0 bis 100°C, gefunden, welches dadurch gekennzeichnet ist, daß man die Reaktion in Gegenwart von 0,1 bis 50 Mol.-% eines N-substituierten Imidazols I, bezogen auf II, durchführt. Als Katalysatoren können im Prinzip beliebige N-substituierte Imidazol-Derivate der Formel I eingesetzt werden. Aus Gründen der einfacheren Aufarbeitung ist es jedoch zweckmäßig, dasjenige N-substituierte Imidazol-Derivat I als Katalysator einzusetzen, welches durch das erfindungsgemäße Verfahren hergestellt werden soll. Die Katalysatormengen liegen im allgemeinen zwischen 0,1 bis 50 Mol.-%, bezogen auf eingesetztes Imidazol II, üblicherweise jedoch zwischen 1 bis 20 Mol.-%.

Die Reaktion wird üblicherweise bei 0 bis 100°C, vorzugsweise 15 bis 75°C, besonders bevorzugt 35 bis 60°C durchgeführt. Zur Erzielung höherer Temperaturen kann auch unter Druck gearbeitet werden.

Als Reaktionsmedium dienen wäßrige Hydroxid-Lösungen, bevorzugt wäßrige Alkalihydroxid-Lösungen, wie Natronlauge, in einer Menge von mehr als 1 Mol Hydroxid-Lösung pro Mol eingesetztes Imidazol II. Man verwendet im allgemeinen Hydroxid-Lösungen mit 10 bis 70 Gew.-% Hydroxid, bevorzugt 30 bis 60 Gew.-%, besonders bevorzugt 50 Gew.-%. Es können auch organische Lösungsmittel zugegeben werden, ohne die Reaktion zu behindern, was aber zur Verminderung der Raum-Zeit-Ausbeute führt.

Als Substituenten R¹ bis R⁴ und Indices n und m in den Formeln I bis III sowie X in Formel III kommen unabhängig voneinander für das Verfahren folgende Bedeutungen in Betracht:
- R¹ -: unverzweigtes oder verzweigtes C₁- bis C₂₀-Alkyl, vorzugsweise unverzweigtes oder verzweigtes C₁- bis C₁₂-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, tert.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Nonyl, n-Decyl, iso-Decyl, n-Undecyl, iso-Un-decyl, n-Dodecyl und iso-Dodecyl,
- -: Allyl, 2-Buten-1-yl, 4-Buten-2-yl, 4-Buten-1-yl, 2-Penten-1-yl, 2,2-Dimethylpenten-1-yl, Propinyl, 1,1-Dimethylpropinyl, 1-Methylpropinyl, 1-Butinyl, 2-Butinyl und 4,4-Dibutyl-2-in-1-yl,
- -: unverzweigtes oder verzweigtes C₂- bis C₂₀-Alkoxyalkyl, bevorzugt unverzweigtes oder verzweigtes C₂- bis C₈-Alkoxy-alkyl, wie Methoxymethyl, Ethoxymethyl, n-Propoxymethyl, iso-Propoxymethyl, n-Butoxymethyl, iso-Butoxymethyl, sec.-Butoxymethyl, tert.-Butoxymethyl, n-Pentoxymethyl, iso-Pentoxymethyl, sec.-Pentoxymethyl, tert.-Pentoxymethyl, neo-Pentoxymethyl, 1,2-Dimethylpropoxymethyl, n-Hexoxymethyl, iso-Hexoxymethyl, sec.-Hexoxymethyl, n-Heptoxymethyl, iso-Heptoxymethyl, Methoxy-1-ethyl, Ethoxy-1-ethyl, n-Propoxy-1-ethyl, iso-Propoxy-1-ethyl, n-Butoxy-1-ethyl, iso-Butoxy-1-ethyl, sec.-Butoxy-1-ethyl, tert.-Butoxy-1-ethyl, n-Pentoxy-1-ethyl, iso-Pentoxy-1-ethyl, sec.-Pentoxyl-ethyl, tert.-Pentoxy-1-ethyl, neo-Pentoxy-1-ethyl, 1,2-Dimethyl-propoxy-1-ethyl, n-Hexoxy-1-ethyl, iso-Hexoxyl-ethyl, sec.-Hexoxy-1-ethyl, Methoxy-2-ethyl, Ethoxy2-ethyl, n-Propoxy-2-ethyl, iso-Propoxy-2-ethyl, n-Butoxy2-ethyl, iso-Butoxy-2-ethyl, sec.-Butoxy-2-ethyl, tert.-Butoxy-2-ethyl, n-Pentoxy-2-ethyl, iso-Pentoxy-2-ethyl, sec.-Pentoxy-2-ethyl, tert.-Pentoxy-2-ethyl, neo-Pentoxy-2-ethyl, 1,2-Dimethyl-propoxy-2-ethyl, n-Hexoxy-2-ethyl, iso-Hexoxy-2-ethyl und sec.-Hexoxy-2-ethyl,
- -: unverzweigtes oder verzweigtes C₃- bis C₂₀-Alkenyloxyalkyl, bevorzugt unverzweigtes oder verzweigtes C₃- bis C₈-Alkenyl-oxyalkyl, wie Vinyloxymethyl, Allyloxymethyl, Vinyloxy-1-ethyl, Allyloxy-1-ethyl, Vinyloxy-2-ethyl und Allyloxy-2-ethyl,
- -: C₃- bis C₁₂-Cycloalkyl, bevorzugt C₃- bis C₈-Cycloalkyl, wie Cyclopropyl, Cyclobutyl, Cyclopentyl, Cyclohexyl, Cycloheptyl und Cyclooctyl,
- -: C₄- bis C₂₀-Cycloalkyl-alkyl, bevorzugt C₄- bis C₈-Cyclo-alkylalkyl, wie Cyclopentyl-methyl, 2-Cyclopentyl-ethyl, 1-Cyclopentyl-ethyl, Cyclohexyl-methyl, 1-Cyclohexylethyl und 2-Cyclohexyl-ethyl,
- -: C₇- bis C₂₀-Arylalkyl, bevorzugt C₇- bis C₁₂-Arylalkyl, wie Benzyl, 1-Phenethyl und 2-Phenethyl,
- -: im Arylteil ein- bis dreifach durch Halogen substituiertes C₇- bis C₂₀-Aryl-alkyl, bevorzugt im Phenylteil ein- bis dreifach durch Fluor oder Chlor C₇- bis C₁₀-Phenyl-alkyl, wie 4-Fluorbenzyl, 4-Chlorbenzyl, 2,4-Dichlorbenzyl und 3,4-Dichlorbenzyl,
- -: im Arylteil ein- bis dreifach durch C₁- bis C₈-Alkyl substituiertes C₇- bis C₂₀-Aryl-alkyl, bevorzugt im Phenylteil ein- bis dreifach durch C₁- bis C₄-Alkyl substituiertes C₇- bis C₁₀-Phenyl-alkyl, besonders bevorzugt im Phenylteil ein- bis dreifach durch C₁- bis C₂-Alkyl substituiertes C₇- bis C₁₀-Phenylalkyl, wie 4-Methylphenyl, 4-Ethylphenyl und 4-Methylphenethyl,
- -: im Arylteil ein- bis dreifach durch C₁- bis C₈-Alkoxy substituiertes C₇- bis C₂₀-Aryl-alkyl, bevorzugt im Phenylteil ein- bis dreifach durch C₁- bis C₄-Alkoxy C₇- bis C₁₀-Phenylalkyl, besonders bevorzugt im Phenylteil ein- bis dreifach durch C₁- bis C₂-Alkoxy substituiertes C₇- bis C₁₀-Phenylalkyl, wie 4-Methoxybenzyl, 4-Ethoxybenzyl und 4-Methoxyphenethyl,
- -: im Arylteil ein- bis dreifach durch C₁- bis C₄-Halogenalkyl, substituiertes C₇- bis C₂₀-Aryl-alkyl, bevorzugt im Phenylteil ein- bis dreifach durch C₁- bis C₂-Fluor- und Chloralkyl substituiertes C₇- bis C₁₀-Phenylalkyl, besonders bevorzugt im Phenylteil ein- bis dreifach durch Trifluormethyl und Trichlormethyl substituiertes C₇-C₁₀-Phenylalkyl, wie 4-Trifluormethylbenzyl und 4-Trichlormethylbenzyl,
- -: im Arylteil ein- bis dreifach durch C₁- bis C₄-Halogenalkoxy substituiertes C₇- bis C₂₀-Aryl-alkyl, bevorzugt im Phenylteil ein- bis dreifach durch C₁- bis C₂-Halogenalkoxy substituiertes C₇- bis C₁₀-Phenyl-alkyl, besonders bevorzugt ein- bis dreifach durch Trifluormethoxy und Trichlormethoxy substituiertes C₇- bis C₁₀-Phenylalkyl, wie 4-Trifluor-methoxybenzyl und 4-Trichlormethoxybenzyl,
- R,R³,R⁴ -: unverzweigtes oder verzweigtes C₁- bis C₂₀-Alkyl, vorzugsweise unverzweigtes oder verzweigtes C₁- bis C₁₂-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, tert.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Nonyl, n-Decyl, iso-Decyl, n-Undecyl, iso-Undecyl, n-Dodecyl und iso-Dodecyl,
- -: unverzweigtes oder verzweigtes C₂- bis C₂₀-Alkoxyalkyl, bevorzugt unverzweigtes oder verzweigtes C₂- bis C₈-Alkoxy-alkyl, wie Methoxymethyl, Ethoxymethyl, n-Propoxymethyl, iso-Propoxymethyl, n-Butoxymethyl, iso-Butoxymethyl, sec.-Butoxymethyl, tert.-Butoxymethyl, n-Pentoxymethyl, iso-Pentoxymethyl, sec.-Pentoxymethyl, tert.-Pentoxymethyl, neo-Pentoxymethyl, 1,2-Dimethylpropoxymethyl, n-Hexoxymethyl, iso-Hexoxymethyl, sec.-Hexoxymethyl, n-Heptoxymethyl, iso-Heptoxymethyl, Methoxy-1-ethyl, Ethoxy-1-ethyl, n-Propoxy-1-ethyl, iso-Propoxy-1-ethyl, n-Butoxy-1-ethyl, iso-Butoxy-1-ethyl, sec.-Butoxy-1-ethyl, tert.-Butoxy-1-ethyl, n-Pentoxy-1-ethyl, iso-Pentoxy-1-ethyl, sec.-Pentoxy-1-ethyl, tert.-Pentoxy-1-ethyl, neo-pentoxy-1-ethyl, 1, 2-Dimethylpropoxy-1-ethyl, n-Hexoxy-1-ethyl, iso-Hexoxy-1-ethyl, sec.-Hexoxy-1-ethyl, Methoxy-2-ethyl, Ethoxy-2-ethyl, n-Propoxy-2-ethyl, iso-Propoxy-2-ethyl, n-Butoxy-2-ethyl, iso-Butoxy-2-ethyl, sec.-Butoxy-2-ethyl, tert.-Butoxy-2-ethyl, n-Pentoxy-2-ethyl, iso-Pentoxy-2-ethyl, sec.-Pentoxy-2-ethyl, tert.-Pentoxy-2-ethyl, neo-Pentoxy-2-ethyl, 1,2-Dimethyl-propoxy-2-ethyl, n-Hexoxy-2-ethyl, iso-Hexoxy-2-ethyl und sec.-Hexoxy-2-ethyl,
- -: C₃- bis C₁₂-Cycloalkyl, bevorzugt C₃- bis C₈-Cycloalkyl, wie Cyclopropyl, Cyclobutyl, Cyclopentyl, Cyclohexyl, Cycloheptyl und Cyclooctyl,
- -: Halogen, wie Fluor, Chlor, Brom und Jod, bevorzugt Chlor und Brom,
- -: Aryl, bevorzugt Phenyl, 1-Naphthyl, 2-Naphthyl, 1-Anthryl, 2-Anthryl und 9-Anthryl, besonders bevorzugt Phenyl,
- -: C₇- bis C₂₀-Arylalkyl, bevorzugt C₇- bis C₁₂-Arylalkyl, wie Benzyl, 1-Phenethyl und 2-Phenethyl,
- -: C₇- bis C₂₀-Alkylaryl, bevorzugt C₇- bis C₁₀-Alkylphenyl, wie 2-Methylphenyl, 3-Methylphenyl, 4-Methylphenyl, 2-Ethylphenyl, 3-Ethylphenyl, 3-Ethylphenyl, 2-n-Propylphenyl, 3-n-Propylphenyl, 4-n-Propylphenyl, 2-iso-Propylphenyl, 3-iso-Propylphenyl, 4-iso-Propylphenyl, 2-n-Butylphenyl, 3-n-Butylphenyl, 4-n-Butylphenyl, 2-iso-Butylphenyl, 3-iso-Butylphenyl, 4-iso-Butylphenyl, 2-sec.-Butylphenyl, 3-sec.-Butylphenyl, 4-sec.-Butylphenyl, 2-tert.-Butylphenyl, 3-tert.-Butylphenyl und 4-tert.-Butylphenyl,
- -: C₇- bis C₂₀-Alkoxyaryl, bevorzugt C₇- bis C₁₀-Alkoxyphenyl, wie 2-Methoxyphenyl, 3-Methoxyphenyl, 4-Methoxyphenyl, 2-Ethoxyphenyl, 3-Ethoxyphenyl, 4-Ethoxyphenyl, 2-n-Prop-oxyphenyl, 3-n-Propoxyphenyl, 4-n-Propoxyphenyl, 2-iso-Propoxyphenyl, 3-iso-Propoxyphenyl, 4-iso-Propoxyphenyl, 2-n-Butoxyphenyl, 3-n-Butoxyphenyl, 4-n-Butoxyphenyl, 2-iso-Butoxyphenyl, 3-iso-Butoxyphenyl, 4-iso-Butoxyphenyl, 2-sec.-Butoxyphenyl, 3-sec.-Butoxyphenyl, 4-sec.-ButoXyphenyl, 2-tert.-Butoxyphenyl, 3-tert.-Butoxyphenyl und 4-tert.-Butoxyphenyl,
- -: zwei- oder dreifach durch C₁- bis C₈-Alkyl substituiertes Aryl, bevorzugt zwei- oder dreifach durch C₁- bis C₄-Alkyl substituiertes Phenyl, wie 2,4-Dimethylphenyl, 3,4-Dimethylphenyl und 3,4,5-Trimethylphenyl,
- -: zwei- oder dreifach durch C₁- bis C₈-Alkoxy substituiertes Aryl, bevorzugt zwei- oder dreifach durch C₁- bis C₄-Alkoxy substituiertes Phenyl, wie 3,4-Dimethoxyphenyl und 3,4,5-Trimethoxyphenyl,
- -: ein- bis dreifach durch C₁- bis C₄-Halogenalkyl substituiertes Aryl, bevorzugt ein- bis dreifach durch C₁- bis C₂-Fluor- und Chloralkyl Phenyl, besonders bevorzugt ein- bis dreifach durch Trifluormethyl und Trichlormethyl, substituiertes Phenyl, wie 4-Trifluormethylphenyl und 4-Trichlormethylphenyl,
- -: ein- bis dreifach durch C₁- bis C₄-Halogenalkoxy substituiertes Aryl, bevorzugt ein- bis dreifach durch C₁- bis C₂-Fluor- und Chloralkoxy Phenyl, besonders bevorzugt ein- bis dreifach durch Trifluormethoxy und Trichlormethoxy substituiertes Phenyl, wie Trifluormethoxyphenyl,
- -: ein- bis dreifach durch Halogen substituiertes Aryl, bevorzugt ein- bis dreifach durch Fluor oder Chlor substituiertes Phenyl, wie 4-Chlorphenyl, 3,4-Dichlorphenyl, 4-Bromphenyl, 4-Fluorphenyl und 4-Fluor-3-chlorphenyl,
- -: C₇- bis C₂₀-Arylalkyl, bevorzugt C₇- bis C₁₀-Phenylalkyl, wie Benzyl, Phenethyl, 1-Phenyl-n-propyl, 2-Phenyl-n-propyl, 3-Phenyl-n-propyl, 1-Phenyl-iso-propyl, 2-Phenyl-iso-propyl, 1-Phenyl-n-butyl, 2-Phenyl-n-butyl, 3-Phenyl-n-butyl, 4-Phenyl-n-butyl, 1-Phenyl-iso-butyl, 2-Phenyl-iso-butyl, 3-Phenyl-iso-butyl, 1-Phenyl-sec.-butyl, 1-Benzyl-n-propyl, 2-Phenyl-sec.-butyl, 3-Phenyl-sec.-butyl und 1,1-Dimethylphenethyl,
- -: im Arylteil ein- bis dreifach durch Halogen substituiertes C₇- bis C₂₀-Aryl-alkyl, bevorzugt im Phenylteil ein- bis dreifach durch Fluor oder Chlor C₇-C₁₀-Phenyl-alkyl, wie 4-Fluorbenzyl, 4-Chlorbenzyl, 2,4-Dichlorbenzyl und 3,4-Dichlorbenzyl,
- -: im Arylteil ein- bis dreifach durch C₁- bis C₈-Alkyl substituiertes C₇- bis C₂₀-Aryl-alkyl, bevorzugt im Phenylteil ein- bis dreifach durch C₁- bis C₄-Alkyl substituiertes C₇- bis C₁₀-Phenyl-alkyl, besonders bevorzugt im Phenylteil ein- bis dreifach durch C₁- bis C₂-Alkyl substituiertes C₇- bis C₁₀-Phenylalkyl, wie 4-Methylphenyl, 4-Ethylphenyl und 4-Methylphenethyl,
- -: im Arylteil ein- bis dreifach durch C₁- bis C₈-Alkoxy substituiertes C₇- bis C₂₀-Aryl-alkyl, bevorzugt im Phenylteil ein- bis dreifach durch C₁- bis C₄-Alkoxy C₇- bis C₁₀-Phenylalkyl, besonders bevorzugt im Phenylteil ein- bis dreifach durch C₁- bis C₂-Alkoxy substituiertes C₇- bis C₁₀-Phenylalkyl, wie 4-Methoxybenzyl, 4-Ethoxybenzyl und 4-Methoxyphenethyl,
- -: im Arylteil ein- bis dreifach durch C₁- bis C₄-Halogenalkyl, substituiertes C₇- bis C₂₀-Aryl-alkyl, bevorzugt im Phenylteil ein- bis dreifach durch C₁- bis C₂-Fluor- und Chloralkyl substituiertes C₇- bis C₁₀-Phenylalkyl, besonders bevorzugt im Phenylteil ein- bis dreifach durch Trifluormethyl und Trichlormethyl substituiertes C₇- bis C₁₀-Phenylalkyl, wie 4-Trifluormethylbenzyl und 4-Trichlormethyl-benzyl,
- -: im Arylteil ein- bis dreifach durch C₁- bis C₄-Halogenalkoxy substituiertes C₇- bis C₂₀-Aryl-alkyl, bevorzugt im Phenylteil ein- bis dreifach durch C₁- bis C₂-Halogenalkoxy substituiertes C₇- bis C₁₀-Phenyl-alkyl, besonders bevorzugt ein- bis dreifach durch Trifluormethoxy und Trichlormethoxy substituiertes C₇- bis C₁₀-Phenylalkyl, wie 4-Trifluormeth-oxybenzyl und 4-Trichlormethoxybenzyl
- -: durch ein, zwei oder drei Phenoxygruppen substituiertes Phenyl,
- -: durch Halogen und C₁- bis C₄-Alkyl zwei- oder dreifach substituiertes Phenyl, wie 2-Methyl-4-chlorphenyl und 3-Methyl-4-fluorphenyl,
- -: durch Halogen und C₁- bis C₄-Alkoxy zwei- oder dreifach substituiertes Phenyl, wie 3-Chlor-4-methoxyphenyl,
- -: durch Halogen und C₁- bis C₄-Halogenalkyl zwei- oder dreifach substituiertes Phenyl, wie 2-Chlor-4-trifluormethylphenyl,
- -: durch Halogen und Phenoxy zwei- oder dreifach substituiertes Phenyl, wie 3-Chlor-4-phenoxyphenyl,
- -: durch C₁- bis C₄-Alkyl und C₁- bis C₄-Alkoxy zwei- oder dreifach substituiertes Phenyl, wie 2-Methyl-4-methoxyphenyl,
- -: durch C₁- bis C₄-Alkyl und C₁- bis C₄-Halogenalkyl zwei- oder dreifach substituiertes Phenyl, wie 3-Methyl-4-trichlormethylphenyl,
- -: durch C₁- bis C₄-Alkyl und Phenoxy zwei- oder dreifach substituiertes Phenyl, wie 2-Methyl-4-phenoxyphenyl,
- -: durch C₁- bis C₄-Alkoxy und C₁- bis C₄-Halogenalkyl zwei- oder dreifach substituiertes Phenyl, wie 3-Trifluormethyl-4-methoxyphenyl,
- -: durch C₁- bis C₄-Alkoxy und Phenoxy zwei- oder dreifach substituiertes Phenyl, wie 3-Methoxy-4-phenoxyphenyl,
- -: durch C₁- bis C₄-Halogenalkyl und Phenoxy zwei- oder dreifach substituiertes Phenyl, wie 3-Trifluormethyl-4-phenoxyphenyl,
- -: durch Halogen, C₁- bis C₄-Alkyl und C₁-C₄- bis Alkoxy dreifach substituiertes Phenyl,
- -: durch Halogen, C₁- bis C₄-Alkyl und C₁- bis C₄-Halogenalkyl dreifach substituiertes Phenyl,
- -: durch Halogen, C₁- bis C₄-Alkyl und Phenoxy dreifach substituiertes Phenyl,
- -: durch Halogen, C₁- bis C₄-Alkoxy und C₁- bis C₄-Halogenalkyl dreifach substituiertes Phenyl,
- -: durch Halogen, C1- bis C₄-Alkoxy und Phenoxy dreifach substituiertes Phenyl,
- -: durch Halogen, C₁- bis C₄-Halogenalkyl und Phenoxy dreifach substituiertes Phenyl,
- -: durch C₁- bis C₄-Alkyl, C₁- bis C₄-Alkoxy und C₁- bis C₄-Halogenalkyl dreifach substituiertes Phenyl,
- -: durch C₁- bis C₄-Alkyl, C₁- bis C₄-Alkoxy und Phenoxy dreifach substituiertes Phenyl,
- -: durch C₁- bis C₄-Alkyl, C₁- bis C₄-Halogenalkyl und Phenoxy dreifach substituiertes Phenyl,
- -: durch C₁- bis C₄-Alkoxy, C₁- bis C₄-Halogenalkyl und Phenoxy dreifach substituiertes Phenyl,
R³ und R⁴ gemeinsam
- -: (CH₂)ₙ, wie CH₂, (CH₂)₂, (CH₂)₃, (CH₂)₄, (CH₂)₅ und (CH₂)₆, bevorzugt (CH₂)₃, (CH₂)₄, (CH₂)₅, (CH₂)₆, besonders bevorzugt (CH₂)₃ und (CH₂)₄,
- -: (CH=CH)ₘ, wie (CH=CH), (CH=CH)₂, (CH=CH)₃, bevorzugt (CH=CH)₂), (CH=CH)₃ besonders bevorzugt (CH=CH)₂,
- n -: 1 bis 6, vorzugsweise 3 bis 6, besonders bevorzugt 3 und 4,
- m -: 1 bis 3, vorzugsweise 2 und 3, besonders bevorzugt 2.

Ein- bis dreifach substituiert bedeutet ein-, zwei- oder dreifach substituiert.
Halogen wie Fluor, Chlor, Brom oder Jod, bevorzugt Chlor oder Brom.

### Beispiel 1

### 1-Methylimidazol

In einem 1-l-Vierhalskolben mit Rührer, Thermometer, Tropftrichter und Rückflußkühler wurden 600 ml 50 %ige Natronlauge, 136 g (2 mol) Imidazol und 16,4 g (0,2 mol) 1-Methylimidazol vorgelegt und 312 g (2,2 mol) Methyliodid unter kräftigem Rühren innerhalb 1,5 h bei 40°C unter Kühlung mit einem Wasserbad zugetropft. Nach beendetem Zutropfen wurde noch 30 Min. bei 40°C gerührt, wobei keine exotherme Nachreaktion zu beobachten war. Zur Aufarbeitung wurde mit Chloroform extrahiert, die vereinigten organischen Phasen mit Wasser gewaschen und über Natriumsulfat getrocknet. Dann wurde das Chloroform im Vakuum entfernt und der Rückstand im Vakuum destilliert. Man erhielt 136,2 g GC-reines 1-Methylimidazol (Ausbeute 119,7 g (73 %) nach Abzug des als Katalysator eingesetzten Produktes) vom Siedepunkt 72 - 73°C bei 13 mbar.

### Beispiel 2

### 1-Ethyl-2-methylimidazol

In einem 1-l-Vierhalskolben mit Rührer, Thermometer, Tropftrichter und Rückflußkühler wurden 450 ml 50 %ige Natronlauge, 123 g (1,5 mol) 2-Methylimidazol und 16,5 g (0,15 mol) 1-Ethyl-2-methylimidazol vorgelegt und 257 g (1,65 mol) Ethyliodid unter kräftigem Rühren bei 40°C zugetropft. Nach beendetem Zutropfen wurde noch 30 Min. bei 40°C nachgerührt, wobei keine exotherme Nachreaktion zu beobachten war. Zur Aufarbeitung wurde mit Chloroform extrahiert, die vereinigten organischen Phasen mit Wasser gewaschen und über Natriumsulfat getrocknet. Dann wurde das Chloroform im Vakuum entfernt und der Rückstand im Vakuum destilliert. Man erhielt 158,5 g GC-reines 1-Ethyl-2-methylimidazol (Ausbeute 142 g (86 %) nach Abzug des als Katalysator eingesetzten Produktes) vom Siedepunkt 88-89°C bei 20 mbar.

### Beispiel 3

### 1-Allylimidazol

In einem 1-l-Vierhalskolben mit Rührer, Thermometer, Tropftrichter und Rückflußkühler wurden 260 ml 50 %ige Natronlauge, 102 g (1,5 mol) Imidazol und 16,2 g (0,15 mol) 1-Allylimidazol vorgelegt und 126,2 g (1,65 mol) Allylchlorid unter kräftigem Rühren innerhalb einer Stunde so zugetropft, daß die Temperatur unter 40°C blieb (gelegentliche Kühlung mit einem Wasserbad). Nach beendetem Zutropfen wurde noch 30 Min. bei 40°C nachgerührt und dann mit 350 g Wasser verdünnt, um die Salze vollständig zu lösen. Anschließend trennte man die organische Phase ab, extrahierte die Wasserphase mit Chloroform und trocknete die vereinigten organischen Phasen über Natriumsulfat. Nach dem Entfernen des Chloroforms wurde im Vakuum destilliert. Man erhielt 165,3 g GC-reines 1-Allylimidazol (Ausbeute 149 g (92 %) nach Abzug des als Katalysator eingesetzten Produktes) vom Siedepunkt 90 - 92°C bei 10 mbar.

### Beispiel 4

### 1-Butylimidazol

In einem 1-l-Vierhalskolben mit Rührer, Thermometer, Tropftrichter und Rückflußkühler wurden 260 ml 50 %ige Natronlauge, 102 g (1,5 mol) Imidazol und 18,6 g (0,15 mol) 1-Butylimidazol vorgelegt und 153 g (1,65 mol) Butylchlorid bei 50°C zugetropft. Nach beendetem Zutropfen wurde noch 2 Stdn. am Rückfluß gekocht und dann mit 350 g Wasser verdünnt, um die Salze vollständig zu lösen. Anschließend trennte man die organische Phase ab, extrahierte die Wasserphase mit Toluol und trocknete die vereinigten organischen Phasen über Natriumsulfat. Nach dem Entfernen des Toluols wurde im Vakuum destilliert. Man erhielt 192 g GC-reines 1-Butylimidazol (Ausbeute 173,4 g (93 %) nach Abzug des als Katalysator eingesetzten Produktes) vom Siedepunkt 78 - 80°C bei 2 mbar.

### Beispiel 5

### 1-Benzylimidazol

In einem 1-l-Vierhalskolben mit Rührer, Thermometer, Tropftrichter und Rückflußkühler wurden 260 ml 50 %ige Natronlauge, 102 g (1,5 mol) Imidazol und 18 g (0,11 mol) 1-Benzylimidazol vorgelegt und 209 g (1,65 mol) Benzylchlorid bei 50°C unter Kühlung mit einem Wasserbad zugetropft. Nach beendetem Zutropfen wurde noch 30 Min. bei 50°C nachgerührt und dann mit 350 g Wasser verdünnt, um die Salze vollständig zu lösen. Anschließend trennte man die organische Phase ab, extrahierte die Wasserphase mit Toluol und trocknete die vereinigten organischen Phasen über Natriumsulfat. Nach dem Entfernen des Toluols wurde im Vakuum destilliert. Man erhielt 243 g 99 %iges 1-Benzylimidazol (Ausbeute 222 g (94 %) nach Abzug des als Katalysator eingesetzten Produktes) vom Siedepunkt 132 -135°C bei 0,5 mbar.

### Beispiel 6

### 1-Benzyl-2-phenyl-imidazol

Zur Mischung von 54 g (0,375 mol) 2-Phenylimidazol, 6,9 g (0,025 mol) 1-Benzyl-2-phenylimidazol, 300 g 50 %iger Natronlauge und 100 ml Toluol tropfte man unter kräftigem Rühren bei 50°C 52,5 g (0,415 mol) Benzylchlorid und rührte noch 5 h bei 50°C. Dann wurde mit Wasser verdünnt, um ausgefallenes Natriumchlorid in Lösung zu bringen. Die organische Phase wurde abgetrennt und die Wasserphase nochmals mit Toluol extrahiert. Nach dem Entfernen des Toluols wurde im Vakuum destilliert. Man erhielt 85 g 99 %iges 1-Benzyl-2-phenyl-imidazol (Ausbeute 78,3 g (89 %) nach Abzug des als Katalysator eingesetzten Produktes) vom Siedepunkt 132 - 135°C bei 0,5 mbar.

### Beispiel 7

### 1-Allylimidazol

Die Umsetzung wurde völlig analog zu Beispiel 3 durchgeführt mit dem Unterschied, daß statt des Allylchlorids 200 g (1,65 mol) Allylbromid eingesetzt wurden. Die Ausbeute betrug 76 %.

Vergleichsbeispiele analog Liebigs Ann. Chem. (1983), 1078-1080.

### Beispiel A

### 1-Methylimidazol

In einem 1-l-Vierhalskolben mit Rührer, Thermometer, Tropftrichter und Rückflußkühler wurden 600 ml 50 %ige Natronlauge und 136 g (2 mol) Imidazol vorgelegt und 312 g (2,2 mol) Methyliodid unter kräftigem Rühren bei 35°C zugetropft. Nach dem Erwärmen auf 40°C setzte nach kurzer Zeit eine exotherme Reaktion ein, in deren Verlauf die Temperatur trotz Kühlung auf 55°C anstieg. Zur Aufarbeitung wurde mit Chloroform extrahiert, die vereinigten organischen Phasen mit Wasser gewaschen und über Natriumsulfat getrocknet. Dann wurde das Chloroform im Vakuum entfernt und der Rückstand im Vakuum destilliert. Man erhielt 87 g (Ausbeute 53 %) GC-reines 1-Methylimidazol vom Siedepunkt 72 - 73°C bei 13 mbar.

### Beispiel B

### 1-Ethyl-2-methylimidazol

In einem 1-l-Vierhalskolben mit Rührer, Thermometer, Tropftrichter und Rückflußkühler wurden 450 ml 50 %ige Natronlauge und 123 g (1,5 mol) 2-Methylimidazol vorgelegt und 257 g (1,65 mol) Ethyliodid unter kräftigem Rühren bei 35°C zugetropft. Wenige Min. nach beendetem Zutropfen setzte eine exotherme Reaktion ein, in deren Verlauf die Temperatur auf 55°C anstieg. Zur Aufarbeitung wurde mit Chloroform extrahiert und die vereinigten organischen Phasen mit Wasser gewaschen und über Natriumsulfat getrocknet. Dann wurde das Chloroform im Vakuum entfernt und der Rückstand im Vakuum destilliert. Man erhielt 68 g (Ausbeute 62 %; Literaturangabe: 73 %) GC-reines 1-Ethyl-2-methylimidazol vom Siedepunkt 88 - 89°C bei 20 mbar.

### Beispiel C

### 1-Allylimidazol

In einem 1-l-Vierhalskolben mit Rührer, Thermometer, Tropftrichter und Rückflußkühler wurden 260 ml 50 %ige Natronlauge und 102 g (1,5 mol) Imidazol vorgelegt und 126,2 g (1,65 mol) Allylchlorid unter kräftigem Rühren bei 40°C zugetropft. 30 Min. nach beendetem Zutropfen setzte eine exotherme Reaktion ein, in deren Verlauf die Temperatur trotz Kühlung mit einem Eisbad auf 60°C anstieg. Zur Aufarbeitung wurde mit 350 g Wasser verdünnt, um die Salze vollständig zu lösen. Anschliepend trennte man die organische Phase ab und extrahierte die Wasserphase mit Chloroform. Die vereinigten organischen Phasen wurden nach dem Entfernen des Chloroforms im Vakuum destilliert. Man erhielt 95 g (Ausbeute 58 %) GC-reines 1-Allylimidazol vom Siedepunkt 90 - 92°C bei 10 mbar.

## Patentansprüche

1. Verfahren,zur Herstellung von N-substituierten Imidazolen der allgemeinen Formel I 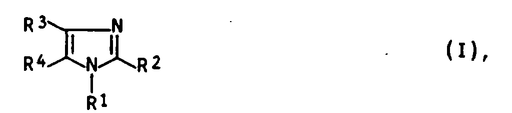 in der die Substituenten
R¹ C₁- bis C₂₀-Alkyl, Allyl, 2-Buten-1-yl, 4-Buten-2-yl, 4-Buten-1-yl, 2-Penten-1-yl, 2,2-Dimethylpenten-1-yl, Propinyl, 1,1-Dimethylpropinyl, 1-Methylpropinyl, 1-Butinyl, 2-Butinyl und 4,4-Dibutyl-2-in-1-yl, C₂- bis C₂₀-Alkoxyalkyl, C₃- bis C₂₀-Alkenyloxyalkyl, C₃- bis C₁₂-Cycloalkyl, C₄- bis C₂₀-CYcloalkyl-alkyl, gegebenenfalls durch C₁- bis C₈-Alkyl, C₁- bis C₈-Alkoxy, Halogen, C₁- bis C₄-Halogenalkyl, C₁- bis C₄-Halogenalkoxy oder Phenoxy substituiertes C₇- bis C₂₀-Arylalkyl,
R,R³,R⁴ unabhängig voneinander Wasserstoff, C₁- bis C₂₀-Alkyl, C₂- bis C₂₀-Alkoxyalkyl, C₃- bis C₁₂-Cycloalkyl, Halogen, gegebenenfalls durch C₁- bis C₈-Alkyl, C₁- bis C₈-Alkoxy, Halogen, C₁- bis C₄-Halogenalkyl, C₁- bis C₄-Halogenalkoxy oder Phenoxy, substituiertes Aryl oder C₇- bis C₂₀-Arylalkyl oder R³ und R⁴ gemeinsam eine gegebenenfalls durch C₁- bis C₈-Alkyl, C₁- bis C₈-Alkoxy und/oder Halogen ein- bis zweifach substituierte (CH₂)ₙ- oder (CH=CH)ₘ-Gruppe, in der n für 1 bis 6 und m für 1 bis 3 steht,
bedeuten, durch Umsetzung von Imidazolen der allgemeinen Formel II 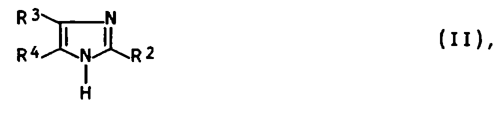 in der R, R³ und R⁴ die obengenannten Bedeutungen haben, mit Halogeniden der allgemeinen Formel III
R¹-X (III),
in der X für Chlor, Brom oder Jod steht und R¹ die obengenannten Bedeutungen hat, in wäßrigen Hydroxid-Lösungen bei Temperaturen von 0 bis 100°C, dadurch gekennzeichnet, daß man die Reaktion in Gegenwart von 0,1 bis 50 Mol.-% eines N-substituierten Imidazols I, bezogen auf II, durchführt.

2. Verfahren nach Anspruch 1, dadurch gekennzeichnet, daß man die Umsetzung in Gegenwart von 1 bis 20 Mol.-% eines N-substituierten Imidazols I, bezogen auf II, durchführt.

3. Verfahren nach Anspruch 1, dadurch gekennzeichnet, daß man die Umsetzung in Gegenwart von 1 bis 20 Mol.-% des herzustellenden N-substituierten Imidazols I, bezogen auf II, durchführt.

4. Verfahren nach Anspruch 1, dadurch gekennzeichnet, daß man die Umsetzung in Gegenwart von 10 bis 70 gew.-%igen wäßrigen Hydroxid-Lösungen durchführt.

5. Verfahren nach Anspruch 1, dadurch gekennzeichnet, daß man als wäßrige Hydroxid-Lösungen wäßrige Alkalihydroxid-Lösungen verwendet.

6. Verfahren nach Anspruch 1, dadurch gekennzeichnet, daß man die Umsetzung bei Temperaturen von 15 bis 75°C durchführt.

7. Verfahren nach Anspruch 1, dadurch gekennzeichnet, daß man als Halogenide III Chloride oder Bromide verwendet.

## Claims

1. A process for preparing N-substituted imidazoles of the formula I 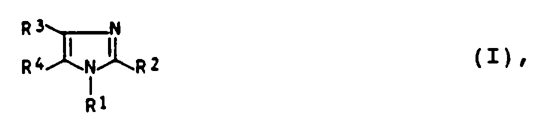 where
R¹ is C₁-C₂₀-alkyl, allyl, 2-butenyl, 1-methyl-2-propenyl, 4-butenyl, 2-pentenyl, 2,2-dimethylpentenyl, propynyl, 1,1-dimethylpropynyl, 1-methylpropynyl, 1-butynyl, 2-butynyl and 4,4-dimethyl-2-butynyl, C₂-C₂₀-alkoxyalkyl, C₃-C₂₀-alkenyloxyalkyl, C₃-C₁₂-cycloalkyl, C₄-C₂₀-cycloalkylalkyl, or C₇-C₂₀-arylalkyl which is unsubstituted or substituted by C₁-C₈-alkyl, C₁-C₈-alkoxy, halogen, C₁-C₄-haloalkyl, C₁-C₄-haloalkoxy or phenoxy,
R, R³ and R⁴ are each, independently of one another, hydrogen, C₁-C₂₀-alkyl, C₂-C₂₀-alkoxyalkyl, C₃-C₁₂-cycloalkyl, halogen, C₇-C₂₀-arylalkyl or aryl which is unsubstituted or substituted by C₁-C₈-alkyl, C₁-C₈-alkoxy, halogen, C₁-C₄,-haloalkyl, C₁-C₄-haloalkoxy or phenoxy, or R³ and R⁴ together form (CH₂)ₙ or (CH=CH)ₘ which is unsubstituted or mono- or disubstituted by C₁-C₈-alkyl, C₁-C₈-alkoxy and/or halogen and in which n is from 1 to 6 and m is from 1 to 3,
by reacting imidazoles of the formula II 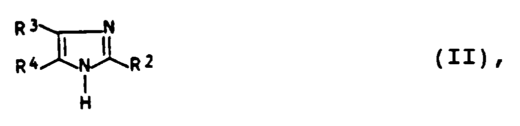 where R, R³ and R⁴ have the abovementioned meanings, with halides of the formula III
R¹-X (III),
where X is chlorine, bromine or iodine, and R¹ has the abovementioned meanings, in aqueous hydroxide solutions at from 0 to 100°C, wherein the reaction is carried out in the presence of from 0.1 to 50 mol % of an N-substituted imidazole I, based on II.

2. A process as claimed in claim 1, wherein the reaction is carried out in the presence of from 1 to 20 mol % of an N-substituted imidazole I, based on II.

3. A process as claimed in claim 1, wherein the reaction is carried out in the presence of from 1 to 20 mol % of the N-substituted imidazole I to be prepared, based on II.

4. A process as claimed in claim 1, wherein the reaction is carried out in the presence of from 10 to 70 % by weight aqueous hydroxide solutions.

5. A process as claimed in claim 1, wherein aqueous alkali metal hydroxide solutions are used as aqueous hydroxide solutions.

6. A process as claimed in claim 1, wherein the reaction is carried out at from 15 to 75°C.

7. A process as claimed in claim 1, wherein chlorides or bromides are used as halides III.

## Revendications

1. Procédé de préparation d'imidazoles N-substitués de la formule générale I 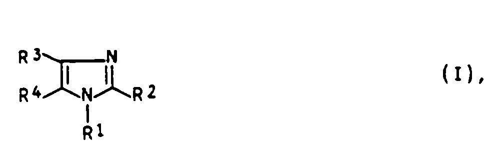 dans laquelle
le symbole R¹
représente un radical alkyle en C₁ à C₂₀, allyle, 2-butène-1-yle, 4-butène-2-yle, 4-butène-1-yle, 2-pentène-1-yle, 2,2-diméthylpentène-1-yle, propynyle, 1,1-diméthylpropynyle, 1-méthylpropynyle, 1-butynyle, 2-butynyle et 4,4-dibutyl-2-yne-1-yle, alcoxyalkyle en C₂ à C₂₀, alcényloxyalkyle en C₃ à C₂₀, cycloalkyle en C₃ à C₁₂, cycloalkylalkyle en C₄ à C₂₀, arylalkyle en C₇ à C₂₀ éventuellement substitué par des radicaux alkyle en C₁ à C₈, alcoxy en C₁ à C₈, des atomes d'halogènes, des radicaux halogénoalkyle en C₁ à C₄, halogénoalcoxy en C₁ à C₄, ou phénoxy,
les symboles R, R³ et R⁴
représentent, chacun indépendamment l'un de l'autre, un atome d'hydrogène, un radical alkyle en C₁ à C₂₀, alcoxyalkyle en C₂ à C₂₀, cycloalkyle en C₃ à C₁₂, des atomes d'halogènes, un radical arylalkyle en C₇ à C₂₀ ou aryle, éventuellement substitué par des radicaux alkyle en C₁ à C₈, alcoxy en C₁ à C₈, des atomes d'halogènes, des radicaux halogénoalkyle en C₁ à C₄ halogénoalcoxy en C₁ à C₄, ou phénoxy, ou bien R³ et R⁴ forment ensemble un radical (CH₂)ₙ ou (CH=CH)ₘ, où n a une valeur de 1 à 6 et m a une valeur de 1 à 3, éventuellement mono ou bisubstitué par des radicaux alkyle en C₁ à C₈, alcoxy en C₁ à C₈ et/ou des atomes d'halogènes,
par la réaction d'imidazoles de la formule générale II 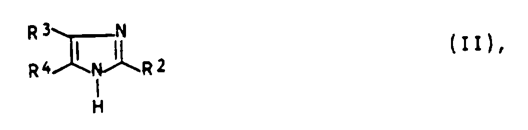
dans laquelle R, R³ et R⁴ possèdent les mêmes significations que celles qui leur ont été attribuées ci-dessus, avec des halogénures de la formule générale III
R¹-X (III),
dans laquelle X représente un atome de chlore, de brome ou d'iode et R¹ possède les significations qui lui ont été attribuées ci-dessus, dans des solutions aqueuses d'hydroxydes, à des températures de 0 à 100°C, caractérisé en ce que l'on entreprend la réaction en présence de 0,1 à 50% molaires d'un imidazole N-substitué I, par rapport à II.

2. Procédé suivant la revendication 1, caractérisé en ce que l'on entreprend la réaction en présence de 1 à 20% molaires d'un imidazole N-substitué I, par rapport à II.

3. Procédé suivant la revendication 1, caractérisé en ce que l'on entreprend la réaction en présence de 1 à 20% molaires de l'imidazole N-substitué à préparer, par rapport à II.

4. Procédé suivant la revendication 1, caractérisé en ce que l'on entreprend la réaction en présence de solutions aqueuses à 10-70% en poids d'hydroxydes.

5. Procédé suivant la revendication 1, caractérisé en ce que l'on utilise des solutions aqueuses d'hydroxydes de métaux alcalins à titre de solutions aqueuses d'hydroxydes.

6. Procédé suivant la revendication 1, caractérisé en ce que l'on entreprend la réaction à des températures de 15 à 75°C.

7. Procédé suivant la revendication 1, caractérisé en ce que l'on utilise des bromures ou des chlorures à titre d'halogénures III.
